# EUROPEAN PATENT APPLICATION

(11) **EP 4 424 302 A1**
(43) Date of publication of application: **04.09.2024**
(21) Application number: 22903257.8
(22) Date of filing: 29.11.2022
(51) Int. Cl.: A61K 9/10, A61K 31/404, A61K 31/44, A61K 45/00, A61K 47/34, A61K 47/36, A61K 47/38, A61K 47/44, A61P 9/10, A61P 27/02, A61P 27/06

(54) **OPHTHALMIC PREPARATION OF TYROSINE KINASE INHIBITOR, AND PREPARATION METHOD THEREFOR AND USE THEREOF**

(30) Priority: 09.12.2021 CN 202111501847
(71) Applicant: Chengdu Ruimu Biopharmaceuticals Co., Ltd., Chengdu, Sichuan 611135 (CN)
(72) Inventor: DONG, Qing, Chengdu, Sichuan 611135 (CN); XUE, Lubing, Chengdu, Sichuan 611135 (CN); TANG, Xin, Chengdu, Sichuan 611135 (CN)
(74) Representative: Altmann Stößel Dick Patentanwälte PartG mbB
(86) International application number: PCT/CN2022/134961
(87) International publication number: WO 2023/103835

(57) **Abstract**

An ophthalmic preparation of a tyrosine kinase inhibitor suitable for eye drop administration is prepared from the following active ingredients and adjuvants in parts by weight: active ingredient: 0.5-1.5 parts of tyrosine kinase inhibitor, wherein the content of the active ingredient in the ophthalmic preparation is 0.1-1 mg/mL; and pharmaceutically acceptable adjuvants: 200-300 parts of the surfactant, 0.5-7.0 parts of the tackifier, 10-800 parts of the solubilizer, with the balance being solvent. The ophthalmic preparation can effectively deliver the active ingredient to the fundus oculi to treat fundus angiogenesis diseases, with high absorption and utilization rate, small particle size and good stability, which are suitable for preparing sterile preparation by means of microporous filter membrane sterilization method and/or high-temperature sterilization method.

## Description

### Field of the invention

The present invention belongs to the field of pharmaceutical preparations, and specifically relates to an ophthalmic preparation of a tyrosine kinase inhibitor, preparation methods therefor and uses thereof.

### Background of the invention

The aging of the population has led to a significant increase in fundus neovascularization diseases (FND) such as cataract, glaucoma, age-related macular degeneration (AMD), and diabetic macular edema (DME). Currently, using the medicaments that have an antagonistic effect on vascular endothelial growth factor (VEGF) to inhibit fundus angiogenesis is one of the main means for treating related neovascularization diseases.

However, due to the complicated physiological structures and barriers in the eyes, it is difficult for medicaments to penetrate the barriers and reach the posterior vitreous body by eye drops, and thereby it is not easy to effectively treat fundus diseases by ocular surface administration in clinical practice. Thus, for a long time, local injection of medicaments has been used to treat fundus diseases. In order to overcome the eye barriers, Bevacizumab, Ranibizumab, Aflibercept, Conbercept, Brolucizumab and other macromolecular biological drugs against vascular endothelial growth factors are administered by vitreous injection to treat fundus diseases caused by neovascularization such as age-related macular degeneration (AMD) and diabetic macular edema (DME). In addition to the above-mentioned macromolecule biological drugs such as anti-VEGF monoclonal antibodies and fusion proteins, small molecule chemical drugs such as tyrosine kinase inhibitors (TKIs) have antagonistic effects on the tyrosine kinase of vascular endothelial growth factor receptor (VEGFR), which can inhibit angiogenesis. TKIs, including axitinib, regorafenib, sunitinib, and nedanib, also have antagonistic effects on platelet-derived growth factor receptor (PDGFR), which can inhibit neovascularization and treat eye diseases related to neovascularization such as AMD and DME (Samanta, et al., Emerging Therapies in Neovascular Age-Related Macular Degeneration in 2020, Asia Pac J Ophthalmol (Phila) 2020; 9: 250-259). In a small preclinical study, TKIaxitinib suspension was injected into the suprachoroidal space of the posterior fundus to treat wAMD, and clinical trials have been initiated in the United States (Kansara VS, Muya LW, Ciulla TA. Evaluation of long-lasting potential of suprachoroidalaxitinib suspension via ocular and systemic disposition in rabbits. Transl Vis Sci Technol. 2021; 10(7): 19).

However, intravitreal or fundus injections belong to invasive administration, and long-term repeated injections lead to the increase in the risk of complications. Furthermore, although tinibs, as small molecule chemical drugs, have better stability than macromolecule biological drugs, their metabolism and distribution speed in the eyeball is faster. To maintain effective drug concentration in fundus lesions, more frequent injections are necessary, that increases the risk of complications and is therefore not suitable for intravitreal injection.

In addition to the above-mentioned local injection in eyes, commonly used administration routes such as oral, injection (non-ocular injection), and other systemic routes increase general toxicity and side effects. Moreover, due to the blood-eye barrier, only a very small amount of drug molecules can reach the posterior segment of the eye, unable to achieve the goal of treating fundus diseases (Zu-Ji Chen, "Practical Ophthalmic Pharmacology", China Science and Technology Press, page 27, 1993). Most of the TKIs (oral anti-tumor drugs) may cause systemic adverse reactions, such as oral administration of axitinib and sorafenib tablets, and their main adverse reactions include diarrhea (with incidence rates of 55% and 53%, respectively), hypertension (40% and 29%), fatigue (39% and 32%), loss of appetite (34% and 29%), nausea (32% and 22%), etc. (Drug instructions, Reference ID: 3078397). In a phase-II clinical trial on the treatment of age-related macular degeneration with oral tinib drugs (X-82), the subjects were administered at different doses of X-82 tablets (50 mg/day, 100 mg/day, 200 mg/day), and after 6 months, visual improvement was non-inferior, but the clinical trial was suspended due to its toxicity (Cohen et al., APEX: a phase II randomised clinical trial evaluating the safety and preliminary efficacy of oral X-82 to treat exudative age-related macular degeneration, Br J Ophthalmol, 2021 May; 105(5): 716-722).

In view of the limitations in the safety and efficacy of oral, injection, ocular local injection, and other routes, there is an urgent need to investigate new delivery ways for TKIs (such as tinib drugs) to treat fundus neovascularization diseases.

Eye drop administration (conjunctival sac administration) is the most convenient and safe routes for ophthalmic drug delivery, and belongs to local administration, with the advantages of small dosage and less toxic side effects. However, the cornea has a multi-layer structure, which can be roughly divided from the outside to the inside: the epithelial layer rich in lipids, the stromal layer rich in aqueous components, and the endothelial layer rich in lipids. Eye-drops first come into contact with the tear layer on the surface of the eye after dropping, and then need to penetrate the epithelial, stromal, and endothelial layers of the cornea before reaching the posterior segment of the eye. During this process, due to the dilution of tears, the ocular surface barrier of the cornea and conjunctiva, and the anatomical positions of the lens and vitreous body, there often are high concentrations of eye-drops in the tissues of the front of eye, that causes side-effects. However, it is still difficult for the eye-drops to enter the posterior segment of the eye with effective therapeutic concentrations. Therefore, although eye drops are safe, it is hard for the pharmaceutical preparations in the prior art to deliver medicaments into the posterior segment of the eye and effectively treat fundus diseases.

In previous research, the inventor has developed nanocrystalline eye-drops be able to successfully deliver TKIs to the fundus with aid of dual/single soluble macromolecular excipients (patent application CN110664757A). However, the solution still has the following issues:
Because eye-drops are solutions administered directly into the eyes to play therapeutic effects, thus must be sterile medical products. In order to achieve the sterile purpose of pharmaceutical liquid preparations, two sterile methods including high-temperature sterilization of the finish product or process sterilization (using a 0.22 µm microporous filter membrane for filtration and sterilization) are commonly used in the finish products preparation, to achieve the goal of finish-product sterility (National Pharmacopoeia Commission, Chinese Pharmacopoeia (2020), Volume 4, China Medical Science Press, Beijing, 2020; Pei-Xue Ling, Ophthalmic Drugs and Preparation Techniques, China Light Industry Press, Beijing, 2010). The structure and properties of nanocrystals are directly affected by the change of temperature during the nanocrystalline solutions sterilization. High sterilization temperature of nanocrystalline solutions as finish product may lead to the dissolution of nanocrystals or changes the important physicochemical properties such as nanocrystalline morphology and particle size, or even the disappearance of nanocrystals, resulting in the appearance of gelatinous substances in the solution. Therefore, these common methods cannot be used for sterilization.

While, for the process sterilization using membrane material with a pore size of 0.22 µm for filtration and sterilization, due to the particle size of the nanocrystalline preparation reaching 300-800 nm, the loss of APIs is very high, that has obviously affected the API content of product. Therefore, there are difficulties in sterilization and decontamination for the industrial production process of the product.

In addition, the APIs absorption and availabilities of the nanocrystalline eye-drops are also low comparatively, and the stability need to be improved, as well.

Therefore, it is of great significance to provide an eye drop of a TKI with the low loss of active ingredient as well as the high absorption and availabilities of APIs after sterilization, in order to treat fundus neovascularization diseases by eye drop administration.

### Summary of the invention

The object of the present invention is to provide a more stable eye drop that can reduce the loss of active ingredients during the processes of filtration sterilization or high-temperature sterilization, and have high fundus absorption and availabilities as well as excellent stability (more stable than nanocrystals) for eye drop administration.

The present invention provides an ophthalmic preparation for eye drop administration, that comprises active substances for treating eye diseases and pharmaceutically acceptable carriers or excipients. The active substances for treating eye diseases are small molecule compounds, such as tyrosine kinases inhibitors (TKIs), which have antagonistic effects on the tyrosine kinase of vascular endothelial growth factor receptor (VEGFR) and/or platelet growth factor receptor (PDGFR). Tinib compounds are drug molecules of TKI class, and the most of their free bases are insoluble in water. To prepare eye-drops based on water and permeable to the posterior segment of the eye, it is necessary to disperse Tinib drugs into aqueous solutions and allow them to be stable. The pharmaceutically acceptable carriers or excipients comprise the following components: surfactants, solubilizers, viscosity enhancers, and solvents. Specifically, the present invention provides an ophthalmic preparation of a TKI, which is prepared from the following raw materials and excipients in parts by weight:
active ingredients: 0.5-1.5 parts of TKIs; the content of active ingredients in the ophthalmic preparation is 0.05-1 mg/mL, and preferably 0.1-1 mg/mL;
pharmaceutically acceptable excipients: 20-300 parts of surfactants, 0.5-70 parts of thickening agents, and 10-800 parts of solubilizers, with the balance being a solvent.

Further, the above ophthalmic preparation is prepared from the following raw materials and excipients in parts by weight:
active ingredients: one part of tyrosine kinase inhibitor;
pharmaceutically acceptable excipients: 25-200 parts of surfactants, 1-60 parts of thickening agents, and 27.5-500 parts of solubilizers, with the balance being a solvent.
preferably, the content of active ingredients in the ophthalmic preparation is 0.05-0.5 mg/mL, and more preferably 0.1-0.5 mg/mL.

Further, the tyrosine kinase inhibitors are tinib active pharmaceutical ingredients or pharmaceutically acceptable salts thereof, and the tinib Active Pharmaceutical Ingredient (API) includes but is not limited to at least one of axitinib, sorafenib, regorafinib, pazopanibb, nintedanib, carbozantinib, lenvatinib, and sunitinib.

Further, the HLB (Hydrophile-Lipophile Balance) values of the above surfactants are >10, and preferably 13-29.

More further, the non-ionic surfactants are at least one of polysorbates (Tweens), dehydrated sorbitol fatty acid esters (Spans), polyoxyethylene fatty acid esters (Myrijs), fatty alcohol-polyoxyethylene ethers (Brijs), or poloxamers.

More further, the surfactants are polysorbates and/or poloxamers.

More further, the surfactants are polysorbates and poloxamers, and the mass ratio of polysorbates and polyxamers is (1-5): 1.

More further, th polysorbate is polysorbate-80.

Further, the thickening agents are at least one of methyl cellulose, hydroxypropyl cellulose, hydroxypropyl methylcellulose, carboxymethyl cellulose or salts thereof, hyaluronic acid or salts thereof, xanthan gum, carbomer, or solid polyethylene glycol (PEG);
the solid PEG is that with a molecular weight of not less than 1000; and preferably PEG 4000 (PEG4K), PEG 5000 (PEG5K), or PEG 6000 (PEG6K); more preferably PEG6K.

More further, the thickening agents are at least one of methyl cellulose, hydroxypropyl cellulose, hydroxypropyl methylcellulose, hyaluronic acid or salts thereof, carboxymethyl cellulose or salts thereof, or solid PEG.

Further, the solubilizers are at least one of liquid PEG, cyclodextrin, hydroxypropyl cyclodextrin, tyloxapol, castor oil polyoxyethylene, and polyoxyethylene hydrogenated castor oil.

More further, the solubilizers are liquid PEG, castor oil polyoxyethylene and/or polyoxyethylene hydrogenated castor oil.

More further, the solubilizers are any one of polyethylene glycol (PEG 300), polyethylene glycol 400 (PEG 400), castor oil polyoxyethylene EL-40, or polyoxyethylene hydrogenated castor oil PEG-60, and the HLB value of castor oil polyoxyethylene EL-40 is 13-14.

Further, the solvent is a polar solvent, and preferably water.

Further, the ophthalmic preparation also contains the following pharmaceutically acceptable excipients in parts by weight: 5-60 parts of emulsion stabilizers.

More further, it also contains the following pharmaceutically acceptable excipients in parts by weight: 6-50 parts of emulsion stabilizers.

More further, the emulsion stabilizers are povidone, hydroxyethyl cellulose, and polyvinyl alcohol.

More further, the emulsion stabilizer is povidone.

More further, said povidone has a weight-average molecular weight of 10000-50000 Dalton.

More further, the above ophthalmic preparation also contains one or more of the following pharmaceutically acceptable excipients: osmotic pressure regulators, pH regulators, preservatives;
the osmotic pressure regulators are any one or more of glucose, sodium chloride, potassium chloride, mannitol, sorbitol, sodium citrate, potassium citrate, and glycerol;
the pH regulators are any one or more of hydrochloric acid, sodium hydroxide, acetic acid or salts thereof, citric acid or salts thereof, fumaric acid, succinic acid, sorbic acid, phosphoric acid, sodium dihydrogen phosphate, disodium hydrogen phosphate, boric acid, borax, tartaric acid or salts thereof;
the preservatives are any one or more of sorbic acid, chlorobutanol, sodium chlorite, sodium perborate, quaternary ammonium salts, parabens, and phenylmercuric nitrate; preferably, the quaternary ammonium salts include benzalkonium chloride, benzalkonium bromide, polyquaternium-1 and/or hexadecyltrimethylammonium bromide, and the parabens include methyl hydroxybenzoate, ethyl hydroxybenzoate, and/or propyl hydroxybenzoate.

Further, the pH value of the above preparation is 5-8.

More further, the pH value of the above preparation is 6-8.

More further, the pH value of the above preparation is 6-7.

The present invention also provides a method for preparing the ophthalmic preparation mentioned above, characterized in that it involves mixing and dispersing the active ingredients and pharmaceutically acceptable excipients, followed by stirring dispersion and/or homogenizing dispersion.

Further, the above preparation method comprises the following steps:
(1) The surfactant is dispersed in the solvent to obtain solution A, while the emulsion stabilizer and the thickening agent are dispersed in the solvent to obtain solution B;
(2) The active ingredient is dispersed in the solubilizer, and then added to solution B obtained in step (1), followed by well dispersing, to obtain solution C;
(3) Solution C is added to solution A and dispersed evenly before high-pressure homogenization, to obtain the ophthalmic preparation.

More further, the dispersion in step (2) and/or step (3) is selected from at least one of mechanical stirring and dispersion, magnetic stirring and dispersion, vortex vibration and dispersion, shear dispersion, grinding and dispersion, and ultrasonic dispersion.

More further, the high-pressure homogenization described in step (3) involves first homogenizing 1-5 cycles at a pressure of not exceeding 800 Bar, and then increasing the pressure to 1300 Bar and homogenizing 10-20 cycles, and then reducing the pressure to below 1000 Bar and homogenizing 1-5 cycles.

(4) The solution obtained in step (3) is adjusted to be isotonic and have a pH value of 5-8, with or without osmotic pressure regulators and/or pH regulators, to which is then added or not added preservatives, to obtain the ophthalmic preparation.

The present invention also provides the use of the ophthalmic preparation mentioned above in the manufacturer of medicaments for treating eye diseases.

Further, the medicaments for treating eye diseases are that for treating ocular surface diseases and/or fundus diseases.

More further, the medicaments for treating fundus diseases are that inhibiting neovascularization.

More further, the medicaments for treating fundus diseases are that for treating any one of age-related macular degeneration (AMD), retinal vein obstructive macular edema (RVO-ME), retinal vein occlusion (RVO), diabetic retinopathy (DR), diabetic macular edema (DME), visual decline caused by choroidal neovascularization (CNC) secondary to pathologic myopia (PM), and neovascular glaucoma (NVG).

More further, the medicaments for treating ocular surface diseases are that for treating ocular surface neovascular diseases.

More further, the medicaments for treating ocular surface diseases are that for treating any one of keratitis; corneal neovascularization caused by mechanical injury, chemical injury and/or biological injury; corneal neovascularization associated with pterygium; corneal neovascularization caused by corneal transplant rejection; and limbal stem cell deficiency.

The beneficial effects of the present invention:
1. The eye drops of the present invention are clear solutions with good stability and significantly reduced particle size (as small as 0.1 µm and smaller), which are suitable for passing through 0.22 µm microporous filter membrane for filtration sterilization or/and high-temperature sterilization (121°C), so as to prepare sterile preparations. After sterilization, the loss of active ingredients is low, and the sterilization does not affect APIs delivering and the absorption in the posterior segment of the eye.
2. Even the dosage of API of this preparation is reduced by 90%, the absorbed amount of API in the vitreous body is equivalent to or even higher than that recorded in the patent application CN110664757A (the concentration of API in the eye drops of the present invention is 0.1-0.5 mg/mL, while the concentration of APIs in the examples of the patent application CN110664757A reaches 1 mg/mL), indicating an increased absorption and availabilities in the fundus of the eye drops according to the present invention;

Moreover, in most cases of eye drop administration, 80-90% of the drug solution is discharged from lacrimal ducts along with the tears or absorbed into the bloodstream through the eyelids and conjunctival blood vessels, that may produce systemic toxicity and side effects. Reducing the total dosage while ensuring the delivery of therapeutic drugs to the fundus of the eyes is beneficial for reducing the whole body toxicity. Therefore, the systemic toxic side-effects of the eye drops according to the present invention are less.

The HLB value, as described in the present invention, is the hydrophile-lipophile balance constant.

The polyethylene glycol 4000 (PEG 4K), as described in the present invention, refers to polyethylene glycol has a weight-average molecular weight of 4000, and so on. The numbers next to other polyethylene glycols represent their weight-average molecular weight.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. Transmission electron microscopy (TEM) photo of the sample prepared in Example 2 (scale = 200 nm).
Figure 2. Transmission electron microscopy (TEM) photo of the sample prepared in Example 3 (scale = 100 nm).

### Examples

The reagents or instruments used in the present invention can be obtained by purchasing those commercially available, and if specific conditions are not indicated, they shall be used according to conventional conditions or manufacturer's recommended conditions.

Some instruments and equipment are as follows:
ES225SM-DR(E) electronic analytical balance, Precisa (Switzerland);
DF-101S Heat-collection type thermostatic magnetic agitator, Gongyi Yingyu Rivet Factory (Henan, China);
WH-2 Mini Vortex meter, Shanghai Huxi Analysis Instrument Factory (Shanghai, China);
Disperse machine: T25 easy clean digital, IKA (Germany);
KQ-500 type ultrasonic cleaning machine, Kunshan Ultrasonic Instruments Co., Ltd (Kunshan, China)
AH-NANO Plus High-Pressure Homogenizer, Antos Nano Technology (Suzhou) Co., Ltd. (China);
PM-DK2 planetary ball mill, Droide Instrument and Equipment (Shanghai) Co., Ltd. (Shanghai, China);
Mettler Toledo FE20 pH meter, Mettler-Toledo (Switzerland);
NS-90 Nanoparticle Size Analyzer, Zhuhai Omec Instrument Co., Ltd. (Zhuhai, China);
Agilent 1100 high performance liquid chromatography (HPLC), Agilent Technologies Inc. (USA);
API4000 Triple Quadrupole Mass Spectrometer (Applied Biosystems, USA);
STY-1A Osmotic Pressure Measuring Instrument, Tiandatianfa Technology Corp., Ltd. (Tianjin, China).

The methods for detecting the properties of the preparations according to the present invention were as follows:

### Method for measuring the particle size:

1 mL of sample prepared in the Example or Comparative Example was transferred to the sample cell. The detection temperature was set to 40 °C, and then the sample cell was put into the NS-90 Nanoparticle Size Analyzer to start the detection. Each sample was tested three times, and the particle size (based on the main particle size distribution and % percentage) and polydispersity index (PdI) were represented with the average of three test results.

### Osmolality measurement:

The molar concentration of the osmotic pressure was determined by measuring the freezing-point depression of the solution. Procedures: the probe of STY-1A osmotic pressure measuring instrument was cleaned. Three portions of 100 µL distilled water were respectively added to three sample tubes, and after preheating the instrument, the sample tube containing 100 µL of distilled water was screwed onto the instrument probe, followed by selecting "clean 3 times" and clicking "Clean", that was repeated three times. Measuring: After filling in the sample information in the instrument information table, click "Testing"; a pipette was used to transfer 100 µL of sample into the tube, which was gently screwed onto the instrument, followed by clicking "Start" for measuring. The test was repeated three times, and then the average of three test results was taken as the result.

### Method for detecting pH value:

The FE20 pH meter was calibrated using pH buffer solutions (pH 4.00, 6.86, and 9.18, respectively). The electrodes were rinsed with pure water, and then excess water was sucked off with fiber-free paper. Subsequently, the electrodes were immersed in the liquid sample to be tested, followed by pressing the reading key to start the testing. After the reading stabilized, the number obtained was the pH value of the sample.

Unless otherwise specified, the method for verifying the result of delivering medicaments to the posterior segment of the eye was as follows:
Experimental instrument and equipment: High performance liquid chromatography, model: LC-20AD (Shimadzu, Japan); mass spectrometer: model: API4000 triple quadrupole mass spectrometer (Applied Biosystems, USA); chromatographic column: Fortis Pace C18 5 µM, 2.1 × 30 mm (Fortis, UK).

Methods for eye testing in rats: Healthy adult Sprague Dawley (SD) rats were divided into a test group and a control group, with 4 eyes in each group. The test group received eye drops of the ophthalmic preparation prepared in the examples of the present invention, at a dosage of 20 µL/eye. After administration, the animals were euthanized at the pre-determined time points, and then the vitreous bodies or/and aqueous humor were quickly collected. After that, the vitreous samples were homogenized and stored at -80 °C.

Methods for sample processing and testing: After homogenizing the rat vitreous sample, 5 µL was taken out, to which was added 45 µL of 70% methanol-water, and then the resultant solution was mixed under ultrasonic for 2 min, followed by vortex mixing for 1 min. Subsequently, 25 µL of internal standard and 150 µL of methanol were successively added. The obtained solution was vortex mixed for 2 min, and centrifuged at 4 °C and 12000 rpm for 10 min. The supernatant was subjected to LC-MS/MS (Positive ion mode, MRM SCAN) analysis. Except for not requiring homogenization, the sample of aqueous humor was processed using the same method, and then analyzed and detected by LC-MS/MS.

Methods for eye testing in rabbits: Healthy adult New Zealand rabbits were selected and divided into a test group and a control group, with 4 eyes in each group. The ophthalmic preparation prepared in the example of the present invention was dropped into the eyes of rabbits in the test group, at a dosage of 50 µL/eye. After administration, the animals were euthanized at the scheduled time points, and then eye tissues (retina, choroid, sclera) were quickly collected. Subsequently, the samples were homogenized and stored at -80 °C.

Methods for sample processing and testing: Referring to the processing and detection methods for tissue samples of rat eyes.

### Example 1: Preparing the ophthalmic preparation according to the present invention

Preparation method: 75 mg of polysorbate-80 (TW-80) was weighed and added to a glass triangular flask containing 10 mL of purified water, and then stirred for 0.5 h with a magnetic stirrer to obtain solution 1; 18 mg of povidone K12 (PVP K12) and 18 mg of hydroxypropyl methyl cellulose (HPMC) were respectively weighed and transferred into a glass triangular flask containing 10 mL of pure water, and then stirred with a magnetic stirrer for 60 min, to obtain solution 2; 2.5 mg of axitinib was weighed and added into a 50 mL polypropylene tube containing 1.2 g of castor oil polyoxyethylene EL-40, to which was added solution 2, and then the resultant solution was stirred for 30 min, followed by adding solution 1. After that, the solution was diluted with water to 25 mL, and stirred for 30 min, to obtain the mixed solution, which was dispersed with a disperser for 3 min at a speed of 10000 rpm, and then allowed to rest until the foam disappeared. Subsequently, the dispersed solution was transferred to a high-pressure homogenizer, and the temperature was controlled to be 15 ± 5 °C. The solution was homogenized under the pressure of about 400 Bar for 3 cycles, then homogenized under the increased pressure of 1300 Bar for 15 cycles, followed by under the reduced pressure of 300 Bar for 2 cycles. The solution was discharged to obtain a homogeneous solution, which was subjected to measuring pH value and the osmotic pressure. The solution was adjusted to pH 7.0 with sodium citrate or/and dilute hydrochloric acid; while the osmotic pressure was adjusted to 272 mOsmol/kg by adding sodium chloride. After preservatives were added, the obtained solution was stirred and dispersed, and then filtered under reduced pressure by passing through 0.45 µm filter membrane, to obtain the product as a solution.

HPLC detection: Agilent HPLC1100 system equipped with a DAD detection unit; HPLC conditions: the chromatographic column was Waters XBridge C18, 5 µm, 4.6 × 250 mm;

Mobile phase A: 0.1% H₃PO₄ solution; mobile phase B: ACN (acetonitrile). Temp.: 35 °C; detection wavelength: 360 nm; flowrate: 0.8 mL/min; gradient elution program: 0': 85% A-15% B, 15': 50% A-50% B, 20-21': 30% A-70% B, 25': 85% A-15% B. The result of content detection by HPLC: 0.078 mg/mL.

The test results of the particle size (main particle sizes and their distribution proportions), Particle size: 13.0 nm (92.1%), PdI: 0.227;

After filtering and sterilizing the solution through a membrane with a pore size of 0.22 µm, the test results of the particle size: Particle size 14.4 nm (100.0%), PdI: 0.080; the test result of the content by HPLC: 0.077 mg/mL.

The experimental results for absorption in rat eyes: 20 µL of the solution was dropped into the rat eyes, and after 0.5 h, the concentration of API in the vitreous body of rats was 19.9 ±3.2 ng/g, while the concentration in aqueous humor was 237.5 ± 47.6 ng/g.

The experimental results for absorption in the tissues of rabbit eyes: 50 µL of the solution was dropped into the New Zealand rabbit eyes, and after 0.5 h, the concentration of API in the retina was 0.92 ± 0.64 ng/g, the concentration in the choroid was 1.22 ± 0.60 ng/g, and the concentration in the sclera was 10.3 ± 5.61 ng/g.

### Example 2: Preparing the ophthalmic preparation according to the present invention

The materials and proportions used are shown in Table 1, and the preparation procedures (adjusting the pH to 6.5) and the content detection were the same as that of Example 1, to obtain the solution; the osmotic pressure was adjusted to 332 mOsmol/kg;

The test results of the particle size, particle size: 12.8 nm (76.0%), PdI: 0.283; the test result for the content by HPLC: 0.181 mg/mL.

After filtering and sterilizing the solution through a membrane with a pore size of 0.22 µm, the test results of the particle size, particle size: 15.2 nm (93.5%), PdI: 0.234; the test result for the content by HPLC: 0.178 mg/mL.

The experimental results for absorption in rat eyes: 20 µL of the solution was dropped into the rat eyes, and after 0.5 h, the concentration of API in the vitreous body of rats was 17.3 ± 9.0 ng/g.

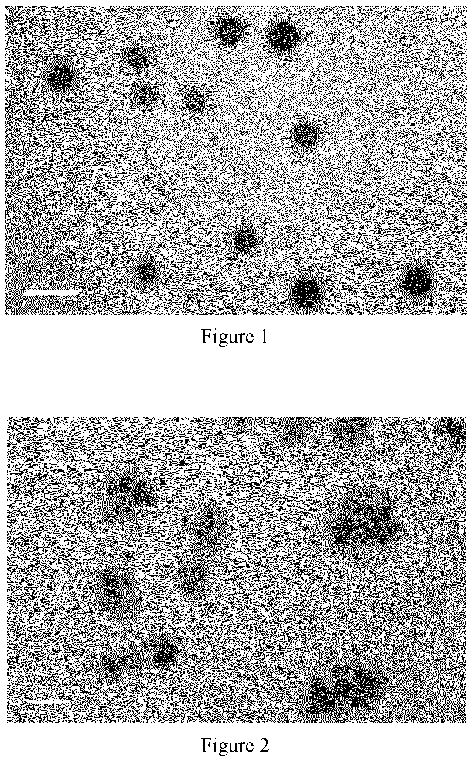

Transmission electron microscopy (TEM) analysis shows that the particle is spherical (Figure 1).

### Example 3: Preparing the ophthalmic preparation according to the present invention

The materials and proportions used are shown in Table 1, and the preparation procedures (adjusting the pH to 5.7) and the content detection were the same as that of Example 1, to obtain the solution; the osmotic pressure was adjusted to 338 mOsmol/kg;

The test results for the particle size: particle size: 13.8 nm (67.2%), 72.6 nm (21.5%), PdI: 0.249; the test result for the content by HPLC: 0.391 mg/mL.

After sterilizing the solution at 121 °C for 20 min, the test results for the particle size: particle size: 12.7 nm (68.6%), 76.7 nm (31.4%), PdI: 0.286; the test result for the content by HPLC: 0.387 mg/mL.

After filtering and sterilizing the solution through a membrane with a pore size of 0.22 µm, the test results for the particle size: particle size: 16.7 nm (70.0%), 126.5 nm (23.7%), PdI: 0.513; the test result for the content by HPLC: 0.389 mg/mL.

The experimental results for absorption in the vitreous body of rats: 20 µL of the solution was dropped into the rat eyes, and after 0.5 h, the concentration of API in the vitreous body of rats was 37.2 ± 25.1 ng/g, while the concentration in aqueous humor was 370 ± 92.4 ng/g.

The experimental results for absorption in the tissues of rabbit eyes: 50 µL of the solution was dropped into the New Zealand rabbit eyes, and after 0.5 h, the concentration of API in the retina was 1.66 ± 2.08 ng/g, the concentration in the choroid was 0.98 ± 0.76 ng/g, and the concentration in the sclera was 9.40 ± 6.41 ng/g.

TEM analysis shows that the particle is snowflake-like (Figure 2)

### Example 4: Preparing the ophthalmic preparation according to the present invention

The materials and proportions used are shown in Table 1, and the preparation procedures (adjusting the pH to 6.1) and the content detection were the same as that of Example 1, to obtain the solution; the osmotic pressure was adjusted to 288 mOsmol/kg;

The test results for the particle size, particle size: 284.6 nm (53.5%), 15.2 nm (46.5%), PdI: 0.500; the test result for the content by HPLC: 0.077 mg/mL.

After filtering and sterilizing the solution through a membrane with a pore size of 0.22 µm, the test results for the particle size: particle size: 12.3 nm (89.8%), PdI: 0.179; the test result for the content by HPLC: 0.076 mg/mL.

The experimental results for absorption in rat eyes: 20 µL of the solution was dropped into the rat eyes, and after 0.5 h, the concentration of API in the vitreous body of rats was 46.8 ± 36.1 ng/g.

### Example 5: Preparing the ophthalmic preparation according to the present invention

The materials and proportions used are shown in Table 1, and the preparation procedures (adjusting the pH to 6.7) and the content detection were the same as that of Example 1, to obtain the solution;

The test results for the particle size, particle size: 106.0 nm (91.2%), PdI: 0.288; the test result for the content by HPLC: 0.081 mg/mL.

After filtering and sterilizing the solution through a membrane with a pore size of 0.22 µm, the test results for the particle size: particle size: 97.8 nm (98.9%), PdI: 0.247; the test result for the content by HPLC: 0.078 mg/mL.

The solution was stored at 40 °C for one month. The appearance and the content of the solution was not obviously changed. The particle size tested: 93.1 nm (99.6%), PdI 0.255; the test result for the content by HPLC: 0.076 mg/mL.

### Example 6: Preparing the ophthalmic preparation according to the present invention

The materials and proportions used are shown in Table 1, and the preparation procedures (adjusting the pH to 6.1) and the content detection were the same as that of Example 1, to obtain the solution;

The test results for the particle size, particle size: 19.8 nm (99.3%), PdI: 0.186; the test result for the content by HPLC: 0.082 mg/mL.

After filtering and sterilizing the solution through a membrane with a pore size of 0.22 µm, the test results for the particle size: particle size: 20.0 nm (98.6%), PdI: 0.218; the test result for the content by HPLC: 0.081 mg/mL.

The solution was stored at 40 °C for one month. The appearance and the content of the solution was not obviously changed. The test results for the particle size: 19.6 nm (99.2%), PdI: 0.236; the test result for the content by HPLC: 0.080 mg/mL.

### Example 7: Preparing the ophthalmic preparation according to the present invention

The materials and proportions used are shown in Table 1, and the preparation procedures (adjusting the pH to 6.3) and the content detection were the same as that of Example 1, to obtain the solution;

The test results for the particle size, particle size: 20.3 nm (95.2%), PdI: 0.246; the test result for the content by HPLC: 0.082 mg/mL.

After filtering and sterilizing the solution through a membrane with a pore size of 0.22 µm, the test results for the particle size, particle size: 19.2 nm (98.3%), PdI: 0.198; the test result for the content by HPLC: 0.081 mg/mL.

The solution was stored at 40 °C for one month. The appearance and the content of the solution was not obviously changed. The test results for the particle size: 18.6 nm (98.8%), PdI: 0.210; the test result for the content by HPLC: 0.081 mg/mL.

### Example 8: Preparing the ophthalmic preparation according to the present invention

The materials and proportions used are shown in Table 1, and the preparation procedures (adjusting the pH to 6.1) and the content detection were the same as that of Example 1, to obtain the solution;

The test results for the particle size, particle size: 13.3 nm (99.4%), PdI: 0.305; the test result for the content by HPLC: 0.083 mg/mL.

After filtering and sterilizing the solution through a membrane with a pore size of 0.22 µm, the test results for the particle size, particle size: 12.7 nm (99.3%), PdI: 0.237; the test result for the content by HPLC: 0.082 mg/mL.

### Example 9: Preparing the ophthalmic preparation according to the present invention

The materials and proportions used are shown in Table 1, and the preparation procedures (adjusting the pH to 6.1) and the content detection were the same as that of Example 1. The osmotic pressure was adjusted to 284 mOsmol/kg by adding sodium chloride and sorbitol, to obtain the solution;

The test results for the particle size, particle size: 15.6 nm (78.2%), PdI: 0.454; the test result for the content by HPLC: 0.084 mg/mL.

After filtering and sterilizing the solution through a membrane with a pore size of 0.22 µm, the test results for the particle size, particle size: 14.3 nm (89.6%), PdI: 0.317; the test result for the content by HPLC: 0.082 mg/mL.

The experimental results for absorption in rat eyes: 20 µL of the solution was dropped into the rat eyes, and after 0.5 h, the concentration of API in the vitreous body of rats was 39.5 ± 26.3 ng/g.

### Example 10: Preparing the ophthalmic preparation according to the present invention

The materials and proportions used are shown in Table 1, and API was regorafenib. The preparation procedures and the content detection were the same as that of Example 1 (The detection wavelength for HPLC: 265 nm), to obtain the solution; the test result for the content: 0.048 mg/mL.

The test results for the particle size, particle size: 17.4 nm (81.2%), PdI: 0.387;

After filtering and sterilizing the solution through a membrane with a pore size of 0.22 µm, the test results for the particle size, particle size: 16.3 nm (91.2%), PdI: 0.342; the test result for the content by HPLC: 0.046 mg/mL.

### Example 11: Preparing the ophthalmic preparation according to the present invention

The materials and proportions used are shown in Table 1, and API was sorafenib tosylate. The preparation procedures and the content detection were the same as that of Example 1 (The detection wavelength for HPLC: 255 nm), to obtain the solution; the test result for the content: 0.089 mg/mL.

The test results for the particle size, particle size: 19.6 nm (88.2%), PdI: 0.406;

After filtering and sterilizing the solution through a membrane with a pore size of 0.22 µm, the test results for the particle size, particle size: 17.9 nm (92.6%), PdI: 0.265; the test result for the content by HPLC: 0.087 mg/mL.

### Comparative example 1.

The materials and proportions used are shown in Table 1, and the preparation procedures and the content detection were the same as that of Example 1, to obtain the solution;

The test results for the particle size, particle size: 2105 nm (100.0%), PdI: 0.645;

After standing at room temperature overnight, the substances in the preparation clumped together and led to the presence of water-oil separation, indicating poor stability of the preparation.

The experimental results for absorption in rat eyes: 20 µL of the solution was dropped into the rat eyes, and after 0.5 h, the concentration of API in the vitreous body of rats was 7.87 ± 2.83 ng/g, indicating that the concentration of the preparation in the fundus was relatively low.

### Comparative example 2.

The materials and proportions used are shown in Table 1. Preparation method: 500 mg of TW-80 and 500 mg of hydroxypropyl cellulose were weighed and added to a triangular flask containing 40 mL of purified water, and then stirred for 30 min, to obtain solution 1; 50 mg of axitinib was weighed and transferred into a 100 mL polypropylene tube, to which was added solution 1, and then the resultant solution was stirred for 10 min, followed by dilution with purified water to 50 mL. The resultant solution was stirred for 30 min, to obtain the mixed solution, which was dispersed with a disperser for 3 min at a speed of 10000 rpm. (1) Using ball milling method: The dispersion was transferred into a ball milling tank, to which was added 80 g of wet zirconium beads (with particle sizes of 0.3-0.4 mm). The polypropylene plastic tube was rinsed with 1 mL of purified water, and then the wash solution was collected into the ball milling tank. Then, the ball milling tank was covered tightly and ground at a speed of 300 rpm for 2 h at 0 °C. After grinding, the resultant solution was filtered with a G2 Buchner funnel, to obtain a milky white suspension.

The test results for the particle size, particle size: 280.9 nm (100.0%), PdI: 0.274; the test result for the content by HPLC: 0.879 mg/mL.

After filtering and sterilizing the solution through a membrane with a pore size of 0.22 µm, the test results for the particle size, particle size: 310.0 nm (100.0%), PdI: 0.248; the test result for the content by HPLC: 0.736 mg/mL.

The solution was sterilized at 121 °C; after 20 min, the layered solution was observed, indicating that this preparation was unstable after high temperature processing.

Alternatively, (2) using high-pressure homogenization, the mixed solution was dispersed at 10000 rpm for 3 min with a disperser. After the machine was stopped and the foam disappeared, the dispersion was transferred to a high-pressure homogenizer, of which the temperature was controlled at 15 ± 5 °C. The solution was homogenized under the pressure of about 400 Bar for 3 cycles, then homogenized under the increased pressure of >1300 Bar for 15 cycles, followed by under the reduced pressure of 300 Bar for 2 cycles, to obtain a homogeneous solution as white suspension.

The test results for the particle size, particle size: 295.0 nm (100.0%), PdI: 0.443; the test result for the content by HPLC: 0.830 mg/mL.

After pressure filtering and sterilizing the solution through a membrane with a pore size of 0.22 µm, the test results for the particle size, particle size: 815.7 nm (80.7%), PdI: 0.736; the test result for the content by HPLC: 0.025 mg/mL.

### Comparative example 3.

The materials and proportions used are shown in Table 1, and the preparation procedures (adjusting the pH to 7.0) were the same as that of comparative example 1, to obtain the solution. After being left at room temperature for 3 days, precipitation was found, indicating that the prepared solution was unstable.

### Comparative example 4.

Eye drops prepared according to No. 12 in Table 8 of patent application CN110664757A: 5 mg of axitinib; 2 mg of Tween 80; 2 mg of HPMC E5, followed by dilution with water to 50 mL. The resultant solution was subjected to high-pressure homogenization. The absorption tests of eye drops obtained was carried out in the vitreous body of rats. 0.5 h after dropping into eyes, the content of API in the vitreous body of rats was 1.7 ng/mL.

### Comparative example 5.

Eye drops prepared according to No. 13 in Table 8 of patent application CN110664757A: 10 mg of axitinib; 2 mg of Tween 80; 2 mg of HPMC E5, followed by dilution with water to 50 mL. The resultant solution was subjected to high-pressure homogenization. The absorption tests of eye drops obtained was carried out in the vitreous body of rats. 0.5 h after dropping into eyes, the content of API in the vitreous body of rats was 6.3 ng/mL.

**Table 1**

| Examp le | Surfactant A | Emulsion stabilizer B | Thickening agent C | Solubilizer D | API amount (mg) E | A/B/C/D/E | Volume (mL) |
|---|---|---|---|---|---|---|---|
| 1 | TW-80 | PVP K12 | HPMC | EL-40 HLB:13-14 | 2.5 | 30/7.2/7.2/480/1 | 25 |
| 2 | TW-80 | PVPK15 | HPMC | PEG-60 hydrogenated castor oil | 6 | 25/6/6/100/1 | 30 |
| 3 | TW-80 | PVP K30 | HPMC | EL-40 HLB:13-14 | 15 | 25/6/6/40/1 | 30 |
| 4 | TW-80 | PVP K17 | HPMC | PEG300 | 4 | 195/50/45/27.5/1 | 40 |
| 5 | TW-80 | | PEG6K +CMC-Na | PEG400 | 3 | 200/0/(50+10)/500/ 1 | 30 |
| 6 | TW-80 + P407 | - | HA | EL-40 HLB:13-14 | 3 | (20+10)/0/1/300/1 | 30 |
| 7 | TW-80 + P407 | - | MC | EL-40 HLB:13-14 | 3 | (20+10)/0/3/300/1 | 30 |
| 8 | TW-20 + P188 | - | HPMC | EL-40 HLB:13-14 | 3 | (50+10)/0/6/100/1 | 30 |
| 9 | TW-80 + P188 | - | HEC | PEG400 | 3 | (17+17)/0/25/333/1 | 30 |
| 10 | TW-80 + P407 | - | MC | EL-40 HLB:13-14 | 2 | (50+10)/0/1/400/1 | 40 |
| 11 | TW-80 + P407 | - | HPMC | PEG-60 hydrogenated castor oil | 3 | (40+10)/0/30/350/1 | 30 |
| Compar ative example 1 | TW-80 | PVP K12 | HPMC | EL-40 HLB:9-10 | 2.5 | 30/7.2/7.2/480/1 | 25 |
| Compar ative example 2 | TW-80 | - | HPC | - | 50 | 10/0/10/0/1 | 50 |
| Compar ative example 3 | - | PVP K12 | HPMC | PEG-60 hydrogenated castor oil | 2 | 0/6/6/500/1 | 20 |

| | | | | | | | |
|---|---|---|---|---|---|---|---|
| Note: the weight-average molecular weight of PVP K12 is 2500 Dalton; the weight-average molecular weight of PVP K15 is 8000 Dalton; the weight-average molecular weight of PVP K17 is 10000 Dalton; and the weight-average molecular weight of PVP K30 is 50000 Dalton. | | | | | | | |

Based on the above results, it was shown that the stability of the eye drops prepared in the examples of the present invention was significantly better than that of the eye drops prepared using weak hydrophilic solubilizers (with HLB values of not greater than 10); moreover, for the eye drops prepared in the examples, at an API dosage equivalent to that of comparative example 1, the absorptive amount in the vitreous body of rats after eye drop administration is higher, that is, the drug availabilites of the eye drops according to the present invention was significantly higher;

The stability of the eye drops prepared in the examples of the present invention was significantly better compared to the preparation without surfactants (comparative example 3);

By comparing the eye drops prepared in the examples of the present invention with that in comparative example 2, the eye drops of the present invention are solutions, with better preparation properties and stability, and the loss of API was lower after sterilization. However, the preparation of comparative example 2 was nanocrystal suspension, and thus there might be a risk of crystal transformation during the production process, which would affect the absorption and distribution of medicaments in the body; there was also a risk of forming clumps during storage, which affected the product quality; moreover, for the suspension of comparative example 2, the loss of API was higher after filtration and sterilization, and the suspension was unstable and layered after high-temperature sterilization, making it difficult to obtain sterile preparations.

Further, by comparing the eye drops prepared in the examples of the present invention with those in comparative examples 4 and 5, it was found that when the API amount of the present invention was equivalent to that of comparative examples (the concentration of axitinib was 0.1 mg/mL), the absorptive amount of axitinib in the vitreous body was significantly higher than that of comparative examples 4 and 5, indicating the availabilities of medicaments from the eye drops of the present invention was obviously improved.

The preparation of the present invention comprised non-ionic surfactants with fixed hydrophilic and lipophilic groups in their chemical structures, and the proportions of their hydrophilic and lipophilic groups determined their HLB values. The lipophilicity or hydrophilicity of the surfactant could be determined by the HLB value (Chapter 3 of Pharmaceutics, edited by Wei-San Pan, Chemical Industry Press, Beijing, 2017; M.R. Shah et al., original work, translated by Ying Liu, Lipid-Based Nanocarriers for Drug Delivery and Diagnosis, Chapter 4, Science Press, Beijing, 2019; S.S. Smail et al., Studies on Surfactants, Cosurfactants, and Oils for Prospective Use in Formulation of Ketorolac Tromethamine Ophthalmic Nanoemulsions, Pharmaceutics 2021, 13, 467). During the research, the present inventors had found that when prepared under the identical or similar conditions, if the HLB value of the surfactant was >10, eye drops containing API had better stability. The surfactants included polysorbate 80 (with a molecular weight of 1130 Dalton), poloxamers 407 (with a molecular weight of 11.5 KDalton), PEG-40 hydrogenated castor oil (with a molecular weight of 2500 Dalton), and solubilizer EL-40 (with a molecular weight of 2500 Dalton), and their HLB values ranged from 13 to 29. By many times experiments, the present inventors had found that using more than one of surfactants with similar HLB values but significant differences in molecular weight resulted in eye drops, which were more stable than that using one surfactant.

Therefore, only on the basis of specific type and amount combinations of excipients in the present invention, the eye drops of the present invention could be obtained, that had excellent stability and high availabilities of APIs, and was suitable for filtration through 0.22 µm microporous filter membranes or/and high-temperature (121 °C) sterilization process.

In summary, the present invention provided an TKIs ophthalmic preparation suitable for eye drop administration, which could effectively deliver active ingredients to the fundus and treat neovascularization diseases. The ophthalmic preparations of the present invention had high absorption and availabilities, with small particle size, good stability, and which are suitable for sterilizations by means of either microporous filtration membrane sterilization or/and high-temperature sterilization methods, with very good clinical application prospects.

## Claims

1. An ophthalmic preparation of a tyrosine kinase inhibitor, **characterized in that** it is prepared from the following active ingredients and excipients in parts by weight:
active ingredients: 0.5-1.5 parts of tyrosine kinase inhibitors; the content of active ingredients in the ophthalmic preparation is 0.05-1 mg/mL;
pharmaceutically acceptable excipients: 20-300 parts of surfactants, 0.5-70 parts of thickening agents, and 10-800 parts of solubilizers, with the balance being a solvent.

2. The ophthalmic preparation according to claim 1, **characterized in that** it is prepared from the following active ingredients and excipients in parts by weight:
active ingredients: 0.5-1.5 parts of tyrosine kinase inhibitors; the content of active ingredients in the ophthalmic preparation is 0.1-1 mg/mL;
pharmaceutically acceptable excipients: 20-300 parts of surfactants, 0.5-70 parts of thickening agents, and 10-800 parts of solubilizers, with the balance being a solvent.

3. The ophthalmic preparation according to claim 1 or 2, **characterized in that** it is prepared from the following active ingredients and excipients in parts by weight:
active ingredients: 1 part of tyrosine kinase inhibitor;
pharmaceutically acceptable excipients: 25-200 parts of surfactants, 1-60 parts of thickening agents, and 27.5-500 parts of solubilizers, with the balance being a solvent; preferably, the content of active ingredients in the ophthalmic preparation is 0.05-0.5 mg/mL, and more preferably 0.1-0.5 mg/mL.

4. The ophthalmic preparation according to any one of claims 1-3, **characterized in that** the tyrosine kinase inhibitors are tinib as active pharmaceutical ingredients (APIs) or pharmaceutically acceptable salts thereof, and the tinib APIs include but are not limited to at least one of axitinib, sorafenib, regorafinib, pazopanib, nintedanib, carbozantinib, lenvatinib, and sunitinib.

5. The ophthalmic preparation according to any one of claims 1-3, **characterized in that** the HLB value of the surfactants is >10, and preferably 13-29.

6. The ophthalmic preparation according to claim 5, **characterized in that** the non-ionic surfactants are at least one of polysorbates, dehydrated sorbitol fatty acid esters, polyoxyethylene fatty acid esters, fatty alcohol-polyoxyethylene ethers, or poloxamers.

7. The ophthalmic preparation according to claim 6, **characterized in that** the surfactants are polysorbates and/or poloxamers.

8. The ophthalmic preparation according to claim 7, **characterized in that** the surfactants are polysorbates and poloxamers, and the mass ratio of polysorbates and polyxamers is (1-5): 1.

9. The ophthalmic preparation according to claim 7 or 8, **characterized in that** said polysorbate is polysorbate-80.

10. The ophthalmic preparation according to any one of claims 1-3, **characterized in that** the thickening agents are at least one of methyl cellulose, hydroxypropyl cellulose, hydroxypropyl methylcellulose, carboxymethyl cellulose or salts thereof, hyaluronic acid or salts thereof, xanthan gum, carbomer, or solid polyethylene glycol (PEG);
the solid PEG has a molecular weight of not less than 1000; and preferably PEG 4000, PEG 5000, or PEG 6000; more preferably PEG 6000.

11. The ophthalmic preparation according to claim 10, **characterized in that** the thickening agents are at least one of methyl cellulose, hydroxypropyl cellulose, hydroxypropyl methylcellulose, hyaluronic acid or salts thereof, carboxymethyl cellulose or salts thereof, carbomer, or solid form PEG.

12. The ophthalmic preparation according to any one of claims 1-3, **characterized in that** the solubilizers are at least one of liquid form PEG, cyclodextrin, hydroxypropyl cyclodextrin, tyloxapol, castor oil polyoxyethylene, and polyoxyethylene hydrogenated castor oil.

13. The ophthalmic preparation according to claim 12, **characterized in that** the solubilizers are liquid form PEG, castor oil polyoxyethylene and/or polyoxyethylene hydrogenated castor oil.

14. The ophthalmic preparation according to claim 13, **characterized in that** the solubilizers are any one of PEG 300, PEG 400, castor oil polyoxyethylene EL-40, or polyoxyethylene hydrogenated castor oil PEG-60, and the HLB value of castor oil polyoxyethylene EL-40 is 13-14.

15. The ophthalmic preparation according to any one of claims 1-3, **characterized in that** the solvent is a polar solvent, and preferably water.

16. The ophthalmic preparation of a tyrosine kinase inhibitor according to any one of claims 1-3, **characterized in that** it also contains the following pharmaceutically acceptable excipients in parts by weight: 5-60 parts of emulsion stabilizers.

17. The ophthalmic preparation of a tyrosine kinase inhibitor according to claim 16, **characterized in that** it also contains the following pharmaceutically acceptable excipients in parts by weight: 6-50 parts of emulsion stabilizers.

18. The ophthalmic preparation according to claim 17, **characterized in that** the emulsion stabilizers are at least one of povidone, hydroxyethyl cellulose, and polyvinyl alcohol.

19. The ophthalmic preparation according to claim 18, **characterized in that** the emulsion stabilizer is povidone.

20. The ophthalmic preparation according to claim 19, **characterized in that** a povidone has a weight-average molecular weight of 3500-50000 Dalton, and preferably has a weight-average molecular weight of 10000-50000 Dalton.

21. The ophthalmic preparation according to any one of claims 1 to 20, **characterized in that** it also contains one or more of the following pharmaceutically acceptable excipients: osmotic pressure regulators, pH regulators, and preservatives;
the osmotic pressure regulators are any one or more of glucose, sodium chloride, potassium chloride, mannitol, sorbitol, sodium citrate, potassium citrate, and glycerol;
the pH regulators are any one or more of hydrochloric acid, sodium hydroxide, acetic acid or salts thereof, citric acid or salts thereof, fumaric acid, succinic acid, sorbic acid, phosphoric acid, sodium dihydrogen phosphate, disodium hydrogen phosphate, boric acid, borax, tartaric acid or salts thereof;
the preservatives are any one or more of sorbic acid, chlorobutanol, sodium chlorite, sodium perborate, quaternary ammonium salts, parabens, and phenylmercuric nitrate; preferably, the quaternary ammonium salts include benzalkonium chloride, benzalkonium bromide, polyquaternium-1 and/or hexadecyltrimethylammonium bromide, and the parabens include methyl hydroxybenzoate, ethyl hydroxybenzoate, and/or propyl hydroxybenzoate.

22. The ophthalmic preparation according to any one of claims 1 to 21, **characterized in that** the pH value of the preparation is 5-8; preferably 6-8; more preferably 6-7.

23. The ophthalmic preparation according to any one of claims 1 to 22, **characterized in that** it is eye drops.

24. A method for preparing the ophthalmic preparation according to any one of claims 1 to 23, **characterized in that** it involves mixing and dispersing the active ingredients and pharmaceutically acceptable excipients, followed by stirring dispersion and/or homogenizing dispersion.

25. A method for preparing the ophthalmic preparation according to claim 24, **characterized in that** it comprises the following steps:
(1) The surfactant is dispersed in the solvent to obtain solution A, while the emulsion stabilizer and the thickening agent are dispersed in the solvent to obtain solution B;
(2) The active ingredient is dispersed in the solubilizer, and then added to solution B obtained in step (1), followed by well dispersing, to obtain solution C;
(3) Solution C is added to solution A and dispersed evenly before high-pressure homogenization;
(4) The solution obtained in step (3) is adjusted to be isotonic and have a pH value of 5-8, with or without osmotic pressure regulators and/or pH regulators, to which is then added or not added preservatives, to obtain the ophthalmic preparation.

26. The preparation method according to claim 25, **characterized in that** the dispersion in step (2) and/or step (3) is selected from at least one of mechanical stirring and dispersion, magnetic stirring and dispersion, vortex vibration and dispersion, shear dispersion, grinding and dispersion, and ultrasonic dispersion.

27. The preparation method according to claim 25, **characterized in that** the high-pressure homogenization described in step (3) involves first homogenizing 1-5 cycles at a pressure of not exceeding 800 Bar, and then increasing the pressure to up to 1300 Bar and homogenizing 5-20 cycles, and then reducing the pressure to below 800 Bar and homogenizing 1-5 cycles.

28. The ophthalmic preparation according to any one of claims 1 to 23 for use in the manufacturer of medicaments for treating eye diseases.

29. The use according to claim 28, **characterized in that** the medicaments for treating eye diseases are that for treating ocular surface diseases and/or fundus diseases.

30. The use according to claim 29, **characterized in that** the medicaments for treating fundus diseases are that inhibiting neovascularization.

31. The use according to claim 30, **characterized in that** the medicaments for treating fundus diseases are that for treating any one of age-related macular degeneration (AMD), retinal vein obstructive macular edema (RVO-ME), retinal vein occlusion (RVO), diabetic retinopathy (DR), diabetic macular edema (DME), visual decline caused by choroidal neovascularization (CNC) secondary to pathologic myopia (PM), and neovascular glaucoma (NVG).

32. The use according to claim 29, **characterized in that** the medicaments for treating ocular surface diseases are that for treating ocular surface neovascular diseases.

33. The use according to claim 32, **characterized in that** the medicaments for treating ocular surface diseases are that for treating any one of keratitis; corneal neovascularization caused by mechanical injury, chemical injury and/or biological injury; corneal neovascularization associated with pterygium; corneal neovascularization caused by corneal transplant rejection; and limbal stem cell deficiency.
